# EUROPÄISCHE PATENTSCHRIFT

(11) **EP 0 986 573 B1**
(45) Veröffentlichungstag und Bekanntmachung des Hinweises auf die Patenterteilung: **09.10.2002**
(21) Anmeldenummer: 98934761.2
(22) Anmeldetag: 20.05.1998
(51) Int. Cl.: C07J 1/00, C07J 31/00, C07J 41/00

(54) **NICHTESTROGENE DERIVATE DES ESTRADIOLS MIT ANTIOXIDATIVER AKTIVITÄT**
NON-ESTROGENIC ESTRADIOL DERIVATIVES WITH AN ANTIOXIDANT EFFECT
DERIVES NON-OESTROGENES D'OESTRADIOL A EFFET ANTIOXYDANT

(30) Priorität: 06.06.1997 DE 19723794
(43) Veröffentlichungstag der Anmeldung: 22.03.2000
(73) Patentinhaber: Jenapharm GmbH & Co. KG, 07745 Jena (DE)
(72) Erfinder: DROESCHER, Peter, D-99425 Weimar (DE); MENZENBACH, Bernd, D-07745 Jena (DE); RÖMER, Wolfgang, D-07749 Jena (DE); SCHNEIDER, Brigitt, D-07745 Jena (DE); ELGER, Walter, D-14195 Berlin (DE); KAUFMANN, Günter, D-07743 Jena (DE)
(74) Vertreter: Leybach, Holger Hugo
(86) Internationale Anmeldenummer: DE9801392
(87) Internationale Veröffentlichungsnummer: WO98055496

(56) Entgegenhaltungen:
- EP-A- 0 688 785
- DE-A- 2 409 991
- DE-A- 4 338 316
- US-A- 3 037 033
- PARVIZI N ET AL: "Catecholestrogens in the brain: neuroendocrine integration" JOURNAL OF STEROID BIOCHEMISTRY, Bd. 19, Nr. 1B, 1983, Seiten 615-618, XP002081763
- E. HECKER ET AL: "Umlagerung von Abkömmlingen des Östra-p-chinols-(10.beta.) in Trifluoroacetanhydrid, ein Beitrag zum Mechanismus der Dienon-Phenol-Umlagerung von p-Chinolen" CHEMISCHE BERICHTE., Bd. 97, Nr. 7, 1964, Seiten 1940-1951, XP002081764 WEINHEIM DE
- J. FREI ET AL: "122. Photochemische Reaktionen. Die Photoisomerisierung von 3-Oxo-delta-1,4-Steroiden in Dioxanlösung Strukturaufklärung der Photoisomeren und Bestimmung der Umlagerungs-Sequenzen" HELVETICA CHIMICA ACTA, Bd. 49, Nr. 3, 1966, Seiten 1049-1105, XP002081765 BASEL CH
- NISHINO Y ET AL: "Comparative evaluation of the dissociation rate between the vaginotropic and uterotropic activities of 1-hydroxy-1,3,5(10)-estrat riene derivatives with natural and unnatural configuration at C8 using ovariectomized mice" STEROIDS., Bd. 28, Nr. 3, September 1976, Seiten 325-337, XP002081766 SAN FRANCISCO US
- SCHWARTZ J A ET AL: "Ligand-mediated modulation of estrogen receptor conformation by estradiol analogs" BIOCHEMISTRY., Bd. 32, Nr. 38, 28. September 1993, Seiten 10109-10115, XP002081767 EASTON, PA US
- D. M. PIATAK ET AL: "Cerium(IV) Oxidation of 4-Methyloestra-1,3,5(10)-trienes" JOURNAL OF THE CHEMICAL SOCIETY, CHEMICAL COMMUNICATIONS, Nr. 14, 21. Juli 1971, Seite 772 XP002081768 LETCHWORTH GB
- ZYDOWSKY T M ET AL: "Preparation and acid-catalyzed rearrangements of a steroidal 1,4-quinol" JOURNAL OF THE CHEMICAL SOCIETY, PERKIN TRANSACTIONS 1., Nr. 8, 1980, Seiten 1679-1681, XP002081769 LETCHWORTH GB
- CAMBIE R C ET AL: "Aromatic steroids. II. Chromium trioxide oxidation of some estra-1,3,5(10)-trienes" JOURNAL OF THE CHEMICAL SOCIETY, SECTION C: ORGANIC CHEMISTRY., Nr. 9, 1969, Seiten 1234-1240, XP002081770 LETCHWORTH GB
- PATENT ABSTRACTS OF JAPAN vol. 015, no. 306 (C-0856), 6. August 1991 & JP 03 115222 A (OYO SEIKAGAKU KENKYUSHO), 16. Mai 1991
- TANG M ET AL: "SUPERIOR AND DISTINCT ANTIOXIDANT EFFECTS OF SELECTED ESTROGEN METABOLITES ON LIPID PEROXIDATION. THE DATA ARE COMPARED WITH THOSEOBTAINED WITH OTHER ESTROGENS, INCLUDING 17ALPHA -DIHYDROEQUILENIN" METABOLISM, CLINICAL AND EXPERIMENTAL, Bd. 45, Nr. 4, April 1996, Seiten 411-414, XP002055052

## Beschreibung

Die Erfindung betrifft neue nichtestrogene Derivate des Estradiols mit antioxidativer Aktivität.

Aus N. Parvizi et al.: "Catecholestrogens in the Brain: Neuroendocrine Integration", Journal of steroid Biochemistry, Bd. 19, 1983, Seiten 615-618, E. Hecker et al.: "Umlagerung von Abkömmlingen des Östra-p-chinols (10β) in Trifluoracetanhydrid, ein Beitrag zum Mechanismus der Dienon-Phenol-Umlagerung von p-Chinolen", Chemische Berichte, Bd. 97, 1964, Seiten 1940-1954, J. Frei et al.: "Photochemische Reaktionen - Die Photoisomerisierung von 3-Oxo-Δ^{1,4}-Steroiden in Dioxanlösung - Strukturaufklärung der Photoisomeren und Bestimmung der Umlagerungs-Sequenzen", Helvetica Chimica Acta, Bd. 49, 1966, Seiten 1049-1105, Y. Nishino et al.: "Comparative evaluation of the dissociation rate between the vaginotropic and uterotropic activities of 1-hydroxy-1,3,5(10)-estratriene derivatives with natural and unnatural configuration at C8 using ovariectomized mice", Steroids, Bd. 28, 1976, Seiten 325-337, J. A. Schwartz et al.: "Ligand-mediated modulation of estrogen receptor conformation by estradiol analogs", Biochemistry, Bd. 32, 1993, Seiten 10109-10115, US-A-3,037,033, DE-A-24 09 991, D. M. Piatak et al.: "Cerium(IV) Oxidation of 4-Methyloestra-1,3,5(10)-trienes", Journal of the Chemical Society, Chemical Communications, 1971, Seite 772, T. M. Zydowsky et al.: "Preparation and acid-catalyzed rearrangements of a steroidal 1,4-quinol", Journal of the Chemical Society, Perkin Transactions 1., Nr. 8,1980, Seiten 1679-1681 und R. C. Cambie et al.: "Aromatic steroids. Part II. Chromium trioxide oxidation of some estra-1,3,5(10)-trienes", Journal of the Chemical Society (C), Organic Chemistry, 1969, Seiten 1234-1240 sind Verbindungen der allgemeinen Formel (I) mit 17β-Konfiguration der 17-Hydroxygruppe bekannt.

Aus DE 43 38 314 C1 ist bekannt, daß Estradiol und seine bekannten Derivate mit phenolischem A-Ring und 17-Hydroxygruppe grundsätzlich antioxidative Aktivität besitzen.
In Abhängigkeit von der Struktur zeigen diese Substanzen eine mehr oder weniger stark ausgeprägte Bindungsaffinität zum Estrogenrezeptor.
Die hohe Affinität des natürlichen 17β-Estradiols (100%) sinkt bei Strukturveränderungen, wie Isomerisierungen oder Derivatisierungen, in der Regel deutlich ab.
Sie beträgt jedoch für 17α-Estradiol noch 23% und selbst für das Enantiomere des natürlichen Estradiols, 8α,9β,14β-Estra-1,3,5(10)-trien-3,17α-diol (ent-Estradiol), noch 8.6%.
Diese Größenordnung ist bei Verabreichung der Substanzen mit dem Ziel, die körpereigene antioxidative Kapazität zu erhöhen, abhängig von Dosis- und Applikationsdauer sowie dem Geschlecht, nicht immer tolerierbar.

Führt man entsprechend DE 43 38 316 A1 einen großen Substituenten am Kohlenstoffatom 17 ein, ist die Bindungsaffinität zwar unter Erhöhung der antioxidativen Aktivität, beispielsweise beim 17α-4'-Dimethylamino-phenylmethyl-estra-1,3,5(10),9(11)-tetraen-3,17-diol, bis auf weniger als 1% senkbar , in vivo wurde jedoch selbst für den entsprechenden 3-Methylether, 3-Methoxy-17α-4'-Dimethylamino-phenylmethylestra-1,3,5(10)-trien-17ol, eine hohe Estrogenität gefunden.

Der Erfindung liegt die Aufgabe zugrunde, neue Derivate des Estradiol mit antioxidativer Aktivität zu finden, die keine estrogene Wirksamkeit besitzen.

Diese Aufgabe wird zum einen durch nichtestrogene Derivate des Estradiols mit antioxidativer Aktivität der allgemeinen Formel la, 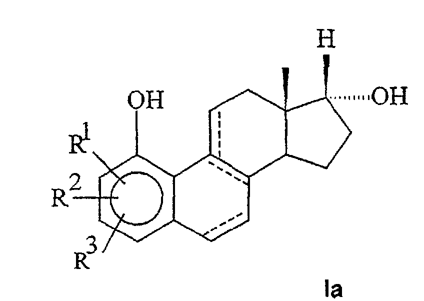
- R¹ =: H, OH
- R², R³ =: H, CH₃
die ein bis zwei Doppelbindungen enthalten kann, wobei die Hydroxygruppen der Estradiolderivate als Ether, Ester oder Sulfamate vorliegen können, gelöst.

Zum anderen wird diese Aufgabe durch nichtestrogene Derivate des 17β-Estradiols mit antioxidativer Aktivität der allgemeinen Formel II, 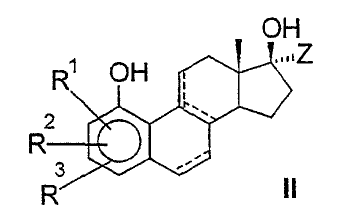
- R¹ =: H, OH
- R², R³ =: H, CH₃
- Z =: (CH₂)ₙAPh,
wobei
n = 0,1
A = Bindung für n = 0,1, O, S, Se für n = 1,
die ein bis zwei Doppelbindungen enthalten kann,
mit einem 17α-Substituenten, der eine Phenylgruppe Ph enthält,
die mit 1 bis 2 Hydroxygruppen und 0 bis 2 Methylgruppen oder mit einer Dimethylaminophenylgruppe und 0 bis 2 Methylgruppen funktionalisiert ist, wobei die Hydroxygruppen der 17β-Estradiolderivate als Ether, Ester oder Sulfamate vorliegen können, gelöst.

Des weiteren betrifft die Erfindung Arzneimittel, enthaltend mindestens ein nichtestrogenes Derivat des 17α-Estradiols oder des 17β-Estradiols sowie die Verwendung der nichtestrogenen Derivate des Estradiols der Formel la und des 17β-Estradiols mit antioxidativer Aktivität der allgemeinen Formel II sowie der nichtestrogenen Derivate des Estradiols mit antioxidativer Aktivität der allgemeinen Formel Ib 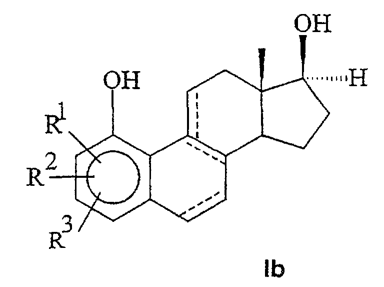
- R¹ =: H, OH
- R², R³ =: H, CH₃,
die ein bis zwei Doppelbindungen enthalten kann,
wobei die Hydroxygruppen der Estradiolderivate als Ether, Ester oder Sulfamate vorliegen können, zur Herstellung eines Arzneimittels zur Prophylaxe und Therapie radikalvermittelter Zellschädigungen, wobei als nichtestrogene Derivate des Estradiols der Formel Ib 4-Methyl-estra-1,3,5(10),6-tetraen-1,17β-diol und 4-Methylestra-1,3,5(10),6,8-pentaen-1,17β-diol bevorzugt verwendet werden.

Bevorzugte Verbindungen sind:
4-Methyl-estra-1,3,5(10)-trien-1,17α-diol
4-Methyl-estra-1,3,5(10),6-tetraen-1,17α-diol
4-Methyl-estra-1,3,5(10),6,8-pentaen-1,17α-diol
17α-4'-Hydroxy-phenylmethyl-4-methyl-estra-1,3,5(10)-trien-1,17β-diol
17α-4'-Hydroxy-phenoxymethyl-4-methyl-estra-1,3,5(10)-trien-1,17β-diol
17α-4'-Hydroxy-thiophenoxymethyl-4-methyl-estra-1,3,5(10)-trien-1,17β-diol
17α-4'-Dimethylamino-phenylmethyl-4-methyl-estra-1,3,5(10)-trien-1,17β-diol
1 7α-3',5'-Dimethyl-4'-hydroxy-phenylmethyl-4-methyl-estra-1,3,5(10)-trien-1 ,17β-diol
17α-3',5'-Dimethyl-4'-hydroxy-phenylmethyl-4-methyl-estra-1,3,5(10),6-tetraen-1,17β-diol
17α-4'-Hydroxy-phenoxymethyl-4-methyl-estra-1,3,5(10),6-tetraen-1,17β-diol

Es wurde nunmehr völlig überraschend gefunden, daß die Verbindungen, Regioisomere des Estradiols und ihre Derivate, die eine phenolische Hydroxygruppe am Kohlenstoffatom 1 und eine Hydroxygruppe am kohlenstoffatom 17 besitzen, weder in vitro noch in vivo estrogen sind - aufgezeigt in Tabelle 1 und 2.
Gleichzeitig besitzen die Verbindungen eine im Vergleich zu Estradiol deutlich gesteigerte antioxidative Aktivität - demonstriert in Tabelle 3 und 4 mittels verschiedenartiger in-vitro-Tests.

Da die Substanzen ein intaktes Steroidgerüst und vergleichbare Polarität zu den natürlichen Estrogenen aufweisen, ist zu erwarten, daß auch die Fähigkeiten zur Überwindung der Blut-Hirn-Schranke und zur Membran-Rezeptor-Wechselwirkung, wie sie in den natürlichen Estrogenen besteht, erhalten bleiben.

Die erfindungsgemäßen bzw. erfindungsgemäß verwendeten Estradiolderivate, bei denen die Estrogenitat unter Verbesserung der antioxidativen Aktivität praktisch vollständig entfernt wurde, sind potentiell zum Einsatz als nichtestrogene Antioxidanzien, insbesondere zur Anwendung bei der postmenopausalen Frau und beim Mann geeignet.
Fehlende Estrogenität ist auch dann von Vorteil, wenn die Hemmung Estrogene generierender Enzyme Ziel einer therapeutischen Strategie ist, beispielsweise die Hemmung von Aromatase und Sulfatase.
Die Enzyme Aromatase und Sulfatase setzen Estron aus Estronsulfat frei.
Von Sulfamaten bisher bekannter phenolischer Steroide ist nach M. J. Reed et al., Steroid sulphatase inhibitor: a new endocrine therapy, Drugs Future 19 (1994), S. 673 und W. Elger et at., Sulfamates of various estrogens are prodrugs with increased systemic and reduced hepatic estrogenicity at oral application, J. Steroid Biochem. Biol., 55 (1995), S. 395-403 eine eine starke Hemmung der Sulfatase bekannt Diese führt in vitro und in vivo zur Hemmung der Freisetzung von Estron aus Estronsulfat.
Die erfindungsgemäßen bzw. erfindungsgemäß verwendeten Verbindungen sind somit auch potentielle Hemmer der Aromatase und Sulfatase.

Aussagen zur Estrogenität der erfindungsgemäßen bzw. erfindungsgemaß verwendeten Verbindungen werden nach Tabelle 1 über die Messung der Estrogen-Rezeptor-Bindung getroffen.

Als Vergleiche zu den erfindungsgemäßen Systemen dienten 17β-Estradiol, 17α-Estradiol, ent-17β-Estradiol (J 855), 3-Methoxy-17α-4'-dimethylamino-phenylmethyl-estra-1,3,5(10)-trien-17ol (J 848) und 17α-4'-dimethylamino-phenylmethyl-estra-1,3,5(10),9(11)-tetraen-3,17-diol (J 844), ebenfalls in Tabelle 1 dargestellt und mit (x) gekennzeichnet.

**Tabelle 1**

| Bindung zum Estrogenrezeptor ausgewählter Verbindungen | |
|---|---|
| Verbindung | relative Bindungsaffinität z. Estrogen-Rezeptor [ % Bindung zum Estrogenrezeptor] |
| 17β-Estradiol (x) | 100 |
| 17α-Estradiol (x) | 22,8 |
| ent-17β-Estradiol - J 855 (x) | 8,6 |
| 4-Methyl-estra-1,3,5(10)-trien-1,17β-diol - J 1178 | 0,04 |
| 4-Methyl-estra-1,3,5(10)-trien-1,17α-diol - J 1179 | < 0,03 |
| 3-Methoxy-17α-4'-dimethylamino-phenylmethyl-estra-1,3,5(10)-trien-17ol - J 848 (x) | 0,7 |
| 17α-4'-dimethylamino-phenylmethyl-estra-1,3,5(10),9(11)-tetraen-3,17-diol - J 844 (x) | 0,7 |

Aus Tabelle 1 ist die nichtestrogene Wirksamkeit in vitro der erfindungsgemäßen bzw. erfindungsgemäß verwendeten Verbindungen im Vergleich zu den Referenzsubstanzen ersichtlich.

Weitere Aussagen zur Estrogenität werden anhand von Ergebnissen in vivo entsprechend dem Allen-Doisy-Test - demonstriert in Tabelle 2 - getroffen.

Tabelle 2 zeigt die Resultate der Estrogenitätsprüfung in vivo an ovarektomierten Ratten.
Estrogene führen bei ovarektomierten Nagern zu charakteristischen Veränderungen am Vaginalepithel. Es kommt zu einer starken Proliferation und Verhornung der oberen Zellagen. Diese Veränderungen sind im Zellbild von Vaginalabstrichen erkennbar.
Das Auftreten kernloser Hornschollen ist Ausdruck einer estrogenspezifischen Wirkung (Allen-Doisy Test).

Als Vergleiche zu den erfindungsgemäßen Systemen dienten 17β-Estradiol, 17α-Estradiol, ent-17β-Estradiol (J 855) und 3-Methoxy-17α-4'-dimethylamino-phenylmethyl-estra-1,3,5(10)-trien-17ol (J 848), ebenfalls in Tabelle 2 dargestellt und mit (x) gekennzeichnet.

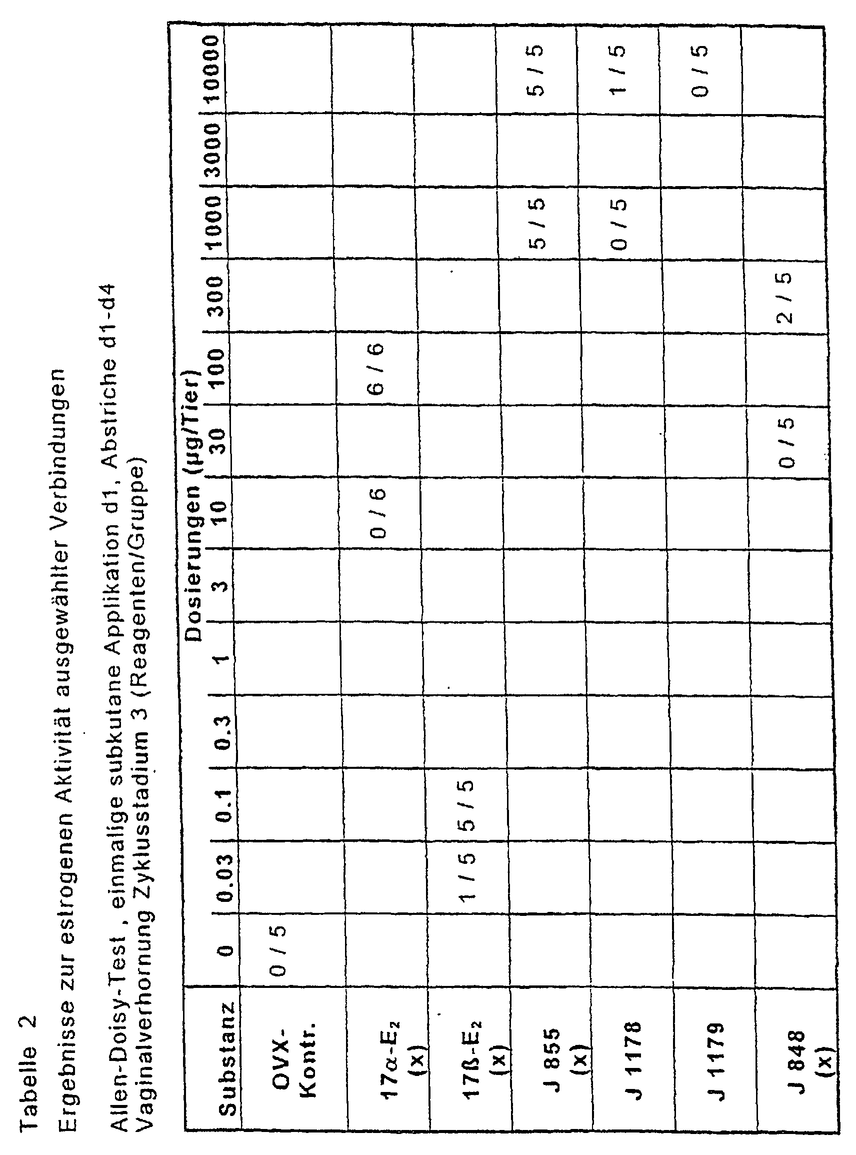

Es ist aus Tabelle 2 ersichtlich, daß die erfindungsgemäßen bzw. erfindungsgemäß verwendeten Verbindungen im Vergleich zu den Referenzsubstanzen selbst bei hohen Dosierungen keine estrogene Wirksamkeit entfalten.

Die antioxidative Aktivität der erfindungsgemäßen bzw. erfindungsgemäß verwendeten Verbindungen wird mittels Aussagen zur Eisen(II)sulfat-katalysierten Lipidperoxidationshemmung in synaptosomalen Membranfraktionen (Ratte) - dargestellt in Tabelle 3 - demonstriert.

Mit der Aussage zu den IC₅₀-Hemmwerten wird die lipidperoxidationshemmende Wirkung der jeweiligen Verbindung charakterisiert.
IC₅₀ gibt die Menge der zuzugebenden Substanz an, um eine 50%ige Hemmung der Lipidperoxidation zu erzielen (Tabelle 3).

Als Vergleiche zu den erfindungsgemäßen bzw. erfindungsgemäß verwendeten Verbindungen dienten 17β-Estradiol, 17α-Estradiol und α-Tocopherol (Vitamin E), Butyrohydroxytoluen (BHT) als Standard, ebenfalls in Tabelle 3 dargestellt und mit (x) gekennzeichnet.

**Tabelle 3**

| Eisen(II)sulfat-katalysierte Lipidperoxidationshemmung ausgewählter Verbindungen | |
|---|---|
| Verbindung | Lipidperoxidationshemmung [IC₅₀ : µMol/l] |
| 17β-Estradiol (x) | 12,40 |
| 17α-Estradiol (x) | 8,9 |
| α-Tocopherol (Vitamin E) (x) | 117,0 |
| 4-Methyl-estra-1,3,5(10)-trien-1,17β-diol - J 1178 | 1,7 |
| 4-Methyl-estra-1,3,5(10)-trien-1,17α-diol - J 1179 | 1,94 |
| Butyrohydroxytoluen (BHT) (x) | 0,95 |

Tabelle 3 verdeutlicht, daß natürliche Estrogene wirksame Inhibitoren der Bildung Thiobarbitursäure-reaktiver Substanzen (TBARS) in Prozessen der mit Fenton's Reagenz induzierten Lipidperoxidation sind. Demnach tragen bereits die antioxidativ wirkenden natürlichen Estrogene 17β-Estradiol und 17α-Estradiol die Fähigkeit, die Lipidperoxidation in synaptosomalen Membran/Lipidfraktionen zu unterdrücken und in deren Konsequenz die Balance verschiedener Redoxprozesse aufrechtzuerhalten.
Es wurde gefunden, daß die Isomeren 4-Methyl-estra-1,3,5(10)-trien-1,17β-diol (J 1178) und 4-Methyl-estra-1,3,5(10)-trien-1,17α-diol (J 1179) diese Wirkung in erheblich stärkerem Maß zeigen.

Weiterhin wird die antioxidative Aktivität der erfindungsgemäßen bzw. erfindungsgemäß verwendeten Verbindungen mittels Aussagen zur Hemmung der Fe(II)-Autooxidation und Stimulierung der Fe(III)-Reduktion - dargestellt in Tabelle 4 - aufgezeigt.

Als Vergleiche zu den erfindungsgemäßen Verbindungen dienten 17β-Estradiol, 17α-Estradiol und Catecholestrogene, ebenfalls in Tabelle 4 dargestellt und mit (x) gekennzeichnet.

**Tabelle 4**

| Hemmung der Fe(II)-Autooxidation und Stimulierung der Fe(III)-Reduktion ausgewählter Verbindungen | | |
|---|---|---|
| Verbindung | Fe(II)-Autooxidation Hemmung [ % ] | Fe(III)-Reduktion Stimulation [ % ] |
| 17β-Estradiol (x) | ≤ 1 | ≤ 1 |
| 17α-Estradiol (x) | ≤ 1 | ≤ 1 |
| 2-Hydroxy-17β-estradiol (x) | 64,20 | 35,26 |
| 4-Hydroxy-17β-esiradiol (x) | 59,51 | 32,71 |
| 4-Methyl-estra-1,3,5(10)-trien-1,17β-diol - J 1178 | 22,26 | 19,23 |
| 4-Methyl-estra-1,3,5(10)-trien-1,17β-diol - J 1179 | 26,07 | 21,41 |

Tabelle 4 veranschaulicht, daß die "klassischen" Estrogene 17β-Estradiol und 17α-Estradiol die getesteten Fe(II)-Autooxidationsprozesse nicht bzw. unwesentlich beeinflussen.
Im Vergleich dazu besitzen Verbindungen, wie die nichtöstrogenen isomeren 4-Methyl-estra-1,3,5(10)-trien-1,17β-diol - J 1178 und 4-Methylestra-1,3,5(10)-trien-1,17β-diol - J 1179 die Fähigkeit, Fe(II)-Autooxidationsprozesse zu hemmen.
Weiterhin ist ersichtlich, daß auch eine Stimuiation der Fe(III)-Reduktion zu Fe(II) stattfindet und mit den Fe(II)-Autooxidationsergebnissen korrelieren.
Die vergleichsweise getesteten Catecholestrogene mit einer Hemmwirkung von ca. 60% zeigen auf, daß die Verbindungen 4-Methyl-estra-1,3,5(10)-trien-1,17β-diol- J 1178 und 4-Methyl-estra-1,3,5(10)-trien-1,17β-diol - J 1179 gut antioxidativ wirksam sind.

Die gegenüber den natürlichen Estrogenen 17β-Estradiol und 17α-Estradiol wesentlich höhere antioxidative Aktivität der erfindungsgemäßen bzw. erfindungsgemäß verwendeten. Verbindungen wurde desweiteren an folgenden Modellen bestätigt:
- Hemmung der Aufnahme oxidativ-modifizierten LDL-Cholesterols in Makrophagen,
- Hemmung der Bildung von Superoxidanionradikalen.

### Biologische Untersuchungsmethoden zur estrogenen und antioxidativen Aktivität der erfindungsgemäßen Verbindungen

### Allen-Doisy-Test

### Zielstellung

Subkutane Prüfung auf estrogene Aktivität.

### Prinzip

Estrogene führen bei ovarektomierten Nagern zu charakteristischen Veränderungen am Vaginalepithel. Es kommt zu einer starken Proliferation und Verhornung der oberen Zellagen. Diese Veränderungen sind im Zellbild von Vaginalabstrichen erkennbar.
Das Auftreten kernloser Hornschollen ist Ausdruck einer estrogenspezifischen Wirkung - Allen-Doisy Test - nach Dorfman R. J., (Hrsg.), Methods in hormone research, S, 72ff, Academic Press New York 1969.

### Tiere

Weibliche Wistarratten (Stamm: Shoe : WIST ≡ Mol : WIST, n = 48) werden im Gewicht von 180 - 200 g angeliefert, randomisiert auf Versuchsgruppen (n = 3-5 Tiere/Gruppe) aufgeteilt, über ca 1 Woche an Haltungsbedingungen adaptiert und anschließend in Ursotaminnarkose ovarektomiert .
- Haltung:: In Makrolonkäfigen Typ M IV in kontrolliert belichteten Räumen (12 Stunden Licht/12 Stunden Dunkelheit).
- Fütterung:: Standardhaltungsdiät für Ratten und Mäuse; Trinkwas ser ad libitum.

### Formulierung und Applikation der Testsubstanzen

Die Prüfsubstanzen zur subkutanen und oralen Applikation in Benzylbenzoat/Ricinusöl (1+4)) bzw. in Myrj (85 mg Myrj in 100 ml 0,9% w/v NaCl-Lösung) werden formuliert.
Das Applikationsvolumen beträgt 0,2 ml/Tier.
- Nach der Eiwaage wird die jeweilige Substanz in Benzylbenzoat durch Behandlung im Ultraschallbad (30 Minuten bei ca 60°C Was sertemperatur) gelöst und anschließend die entsprechende Menge Ricinusöl zugegeben.
- Nach der Eiwaage wird in Myrj suspendiert (unter Zugabe einer geringen Menge Zirkoniumkügelchen) durch Behandlung im Ultra schallbad (30 Minuten bei ca 60°C Wassertemperatur).

### Versuchsdurchführung

Ratten mit einem Gewicht von ca 200 g werden in Ursotaminnarkose ovarektomiert, ca 2 Wochen danach erfolgt die Untersuchung der Tiere auf das Vorliegen eines Kastratenvaginalzellbildes (Dioestrus). Danach wird einmalig subkutan appliziert (d1), Applikationsvolumen 0,2ml/Tier. 24, 48, 54 und 72 Stunden nach der einmaligen Substanzapplikation sind Vaginalabstriche abzunehmen und hinsichtlich Oestrusstadien (Dioestrus = 1; Prooestrus = 2; Oestrus = 3; Metoestrus = 4) mikroskopisch auszuwerten.
Die Untersuchung entsprechend dem standardisierten ALLEN-DOISY Test ist nach 4 Tagen beendet. Die Tiere werden in Aethernarkose durch Dislokation der Halswirbelsäule getötet, der Uterus präpariert und die Feuchtgewichte (Uterus ohne Sekret) ermittelt. Weiterhin erfolgt die Präparation und Gewichtsermittlung der Nebennieren.

### Auswertung

Die Ergebnisse der kolpotropen Prüfung - das Auftreten oestrischer Vaginalzellbilder als Reagenten pro Dosisgruppe sowie die uterotrope Aktivität (Uterusgewichte) werden erfaßt. Die Mittelwerte der Uterusgewichte der Versuchsgruppen sind mit dem Mittelwert der Vehikelgruppe zu vergleichen und gegebenenfalls auf signifikanten Unterschied mit dem t-Test nach STUDENT zu prüfen. Die Feuchtgewichte der Nebennieren aller Gruppen werden ermittelt .

### Eisen(II)sulfat-katalysierte Lipidperoxidationshemmung in synaptosomalen Membranfraktionen (Ratte)

### Material und Methodik

Die Testung der Substanzen auf die Lipidperoxidations-Hemmwirkung nach Braughler JM. et al., The 21-Aminosteroids: Potent inhibitors of lipid peroxidation for the treatment of central nervous system trauma and ischemia, Drugs Future 14 (1989), S.141-152 und nach Buege A. et al., Microsomal lipid peroxidation, Methods Enzymol 52 (1978), S.302-310, mittels des Malondialdehyd/Thiobarbitursäure-Assays, wie nachfolgend, durchgeführt:
**Materialien**
   17β-Estradiol (Jenapharm GmbH), 17α-Estradiol (Sigma Chemicals), α-Tocopherol (Sigma Chemicals), Thiobarbitursäure (TBA; Fluka), Eisen(II)sulfat (Serva).
   Alle anderen Biochemikalien sind von höchster analytischer Reinheit.
**Reaktionsansatz**
   1 ml biologische Probe (enthält 0,1 mg Plasmamembranen), incl. Fenton's Reagenz und Drug. Die 1 ml Gesamtvolumen teilen sich auf in: 0,01 ml synaptosomale Membranfraktion; 0,1 ml Eisen(11)-sulfat (2 mM); 0,1 ml Wasserstoffperoxid (2 mM); bis zu 0,5 ml Testsubstanzlösung und einer anteiligen Menge an 0,9 %iger NaCl-Lösung (nicht PBS) zum Auffüllen auf 1 ml Gesamtvolumen. Der Reaktionsansatz mit und ohne Testsubstanz enthält in jedem Fall 10 % Ethanol (total) als Vehikel.
**Prozedere**
   Der Reaktionsansatz wird 30 min bei 37°C inkubiert, anschließend mit 2 ml Reagenz A abgestoppt und 10 min bei konstanten 80°C inkubiert. Nach dem Abkühlen in einem Eisbad (10 min) wird die Probe in einer Kühlzentrifuge zentrifugiert (1.000 x g; 4°C). Der Überstand wird (bis zu 2 Std. stabil) bei 535 nm gegen den Blindwert gemessen, der bis auf die Membranfraktion alle Reagenzien enthält.
   Als Vergleichswert dient der Ansatz, der neben der Membranfraktion Fenton's Reagenz und 10 % des Vehikels Ethanol enthält.
   Zusammensetzung des Reagenz A:
   15 % (w/v) Trichloressigsäure (15 g); 0,375 % (w/v) Thiobarbitursäure (375 mg); 0,25 N HCI (2,11 ml konz. HCl) in 100 ml wäßriger Lösung.
   Die Testsubstanzen werden vorzugsweise in 95 %igem Ethanol in Form 20 millimolarer Stammlösungen (Haltbarkeit bei minus 20°C, stabil über den Zeitraum von 3 Monaten) angesetzt und unmittelbar vor Versuchsbeginn entsprechend verdünnt. Geprüft wird im Dosierungsbereich 0,1 bis 150 uM.
   In allen Versuchsansätzen wird eine entsprechende Standardsubstanz mitgeführt.
**Bewertungsparameter**
   - Dosis-Wirkungsanalyse der Test- und Standardsubstanzen.
   - Ermittlung der Lipidperoxidationshemmwerte mit mindestens fünf Substanzkonzentrationen im Hemmbereich 30 bis 70 %, bezogen auf die Testwerte mit Vehikel (vorzugsweise Ethanol) und ohne Substanzeffekt.
   - Die Bewertung der Substanzen wird in IC₅₀-Werten (mikromolare Testkonzentrationen bei 50 % Hemmung der Eisen(II)-katalysierten Lipidperoxidation) ausgewiesen.

### Hemmung der Fe(II)-Autooxidation und Stimulierung der Fe(III)-Reduktion

### 1. Fe(II)-Oxidations-Assay

### Material und Methodik

Die Testung der Substanzen auf die Fe(ll)-Autooxidation-Hemmwirkung erfolgt nach Ruiz-Larrea B. et al., Antioxidant effects of estradiol and 2-hydroxyestradiol on iron-induced lipid peroxidation of rat liver microsomes. Steroids 39 (1994), S. 383-388 und Ruiz-Larrea B. et al., Effects of estrogens on the redox chemistry of iron: A possible mechanism of the antioxidant action of estrogens, Steroids 60 (1995), S. 780-783.
Alle Autooxidations-Experimente der Fe(II)-lonen wurden in wäßrigen Lösungen ausgeführt, die synaptosomal Membrane/Lipidfraktionen enthaiten. 1.0 ml biologische Probe, enthaltend 50 µM Fe(II)sulfat, 45 mM Tris-HCl-Puffer (pH 7.4) und 0.08 mg des synaptosomalen Proteins wurde mit den in Ethanol (10% v/v) gelösten Testsubstanzen für 10 min in einem Wasserbad bei 37°C inkubiert, anschließend durch Zugabe von 50 µL einer 0,32 M 1,10-Phenanthrolin-Lösung gestoppt und die Extinktion des Fe(II)-Phenanthroline-Komplexes bei 510 nm gemessen.

### Materialien

17β-Estradiol (Jenapharm GmbH), 17α-Estradiol (Sigma Chemicals), 2-Hydroxy-17β-estradiol und 4-Hydroxy-17β-estradiol (Sigma Chemicals), Thiobarbitursäure (TBA; Fluka), Eisen(11)sulfat (Serva).
Alle anderen Biochemikalien sind von höchster analytischer Reinheit.
Die Testsubstanzen werden vorzugsweise in 95 %igem Ethanol in Form 20 millimolarer Stammlösungen angesetzt und unmittelbar vor Versuchsbeginn entsprechend verdünnt. Geprüft wird im Dosierungsbereich 0,1 bis 150 µM.
In allen Versuchsansätzen wird eine entsprechende Standardsubstanz mitgeführt.

### Bewertungsparameter

- Dosis-Wirkungsanalyse der Test- und Standardsubstanzen.

### 2. Fe(III)-Reduktions-Assay

Die Testung der Substanzen auf die Stimulierung der Fe(III)-Reduktion erfolgte nach Ruiz-Larrea B. et al., Antioxidant effects of estradiol and 2-hydroxyestradiol on iron-induced lipid peroxidation of rat liver microsomes. Steroids 39 (1994), S, 383-388 und Ruiz-Larrea B. et al., Effects of estrogens on the redox chemistry of iron; A possible mechanism of the antioxidant action of estrogens, Steroids 60 (1995), S. 780-783.
Die biologische Probe enthält folgende Komponenten: 25 µM Fe(III)chlorid in 150 mM Tris-HCl (pH 7.4), 15 mM 1,10-Phenanthroline und die zu testende Prüfsubstanz, gelöst im Vehikel Ethanol. Die Bildung des Fe(II)-Phenanthrolin-Komplexes wird spektrophotometrisch bei 510 nm registriert.

### Hemmung der Aufnahme oxidativ-modifizierten LDL-Cholesterols in Makrophagen

Die Messung der Aufnahme von oxidativ-modifiziertem LDL in murinen Makrophagen und Blutmakrophagen menschlichen Ursprungs wurde mittels der Zellkulturmethode nach Fisher M. et al, A 21-aminosteroid inhibits oxidation of human low density lipoprotein by human monocytes and copper, Atherosclerosis 90 (1991), S. 197-202 bzw. nach Leake D.S. und Rankin, S.M., The oxidative modification of Low-Density Lipoproteins by macrophages, Biochem J. 270 (1990), S. 741-748 ermittelt.

### Hemmung der Bildung von Superoxidanionradikalen

Die Messung der Xanthinoxidase-hemmenden Wirkung bei Verwendung von Linolensäure als Bildner für Superoxidanionradikale wird nach Laihia J.K. et al., Lucigenin and linoleate enhanced chemiluminescent assay for superoxide dismutase activity, Free Rad. Biol. Med. 14 (1993) S. 457-461 mittels einer Lucigenin/Luminol-verstärkten
Xanthin/Xanthinoxidase-abhängigen Chemilumineszenzreaktion ermittelt.

## Patentansprüche

1. Nichtestrogene Derivate des Estradiols mit antioxidativer Aktivität der allgemeinen Formel Ia, 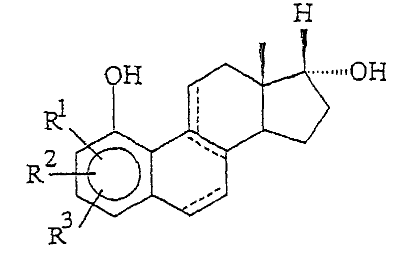
R¹ = H, OH
R², R³ = H, CH₃,
die ein bis zwei Doppelbindungen enthalten kann,
wobei die Hydroxygruppen der Estradioiderivate als Ether, Ester oder Sulfamate vorliegen können.

2. Nichtestrogene Derivate des Estradiols nach Anspruch 1, nämlich 4-Methyl-estra-1,3,5(10)-trien-1,17α-diol, 4-Methyl-estra-1,3,5(10),6-tetraen-1,17α-diol und 4-Methyl-estra-1,3,5(10),6,8-pentaen-1,17α-diol.

3. Nichtestrogene Derivate des 17β-Estradiols mit antioxidativer Aktivität der allgemeinen Formel II, 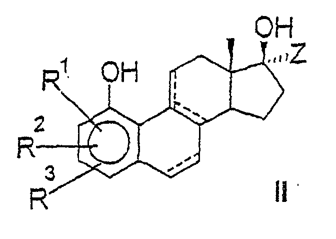
R¹ = H, OH
R², R³ = H, CH₃
Z = (CH₂)ₙAPh,
wobei
n = 0,1
A = Bindung für n = 0,1, O, S, Se für n = 1,
die ein bis zwei Doppelbindungen enthalten kann,
mit einem 17α-Substituenten, der eine Phenylgruppe Ph enthält,
die mit 1 bis 2 Hydroxygruppen und 0 bis 2 Methylgruppen oder mit einer Dimethylaminophenylgruppe und 0 bis 2 Methylgruppen funktionalisiert ist,
wobei die Hydroxygruppen der 17β-Estradiolderivate als Ether, Ester oder Sulfamate vorliegen können.

4. Nichtestrogene Derivate des 17β-Estradiols nach Anspruch 3, nämlich 17α-4'-Hydroxy-phenylmethyl-4-methyl-estra-1,3,5(10)-trien-1,17β-diol, 17α-4'-Hydroxy-phenoxymethyl-4-methyl-estra-1,3,5(10)-trien-1,17β-diol, 17α-4'-Hydroxy-thiophenoxymethyl-4-methyl-estra-1,3,5(10)-trien-1,17β-diol, 17α-4'-Dimethylaminophenylmethyl-4-methyl-estra-1,3,5(10)-trien-1,17β-diol, 17α-3',5'-Dimethyl-4'-hydroxy-phenylmethyl-4-methyl-estra-1,3,5(10)-trien-1,17β-diol, 17α-3',5'-Dimethyl-4'-hydroxy-phenylmethyl-4-methylestra-1,3,5(10),6-tetraen-1,17β-diol und 17α-4'-Hydroxyphenoxymethyl-4-methyl-estra-1,3,5(10),6-tetraen-1,17β-diol.

5. Verwendung der nichtestrogenen Derivate des Estradiols und des 17β-Estradiols mit antioxidativer Aktivität nach einem der Ansprüche 1 bis 4 sowie der nichtestrogenen Derivate des Estradiols mit antioxidativer Aktivität der allgemeinen Formel Ib 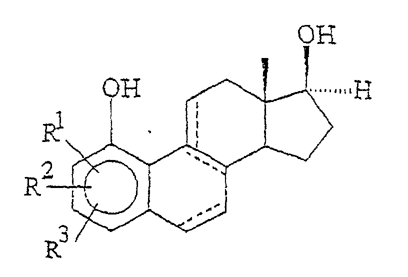
R¹ = H, OH
R², R³ = H, CH₃,
die ein bis zwei Doppelbindungen enthalten kann,
wobei die Hydroxygruppen der Estradiolderivate als Ether, Ester oder Sulfamate vorliegen können, zur Herstellung eines Arzneimittels zur Prophylaxe und Therapie radikalvermittelter Zellschädigungen.

6. Verwendung nach Anspruch 5, wobei als nichtestrogene Derivate des Estradiols 4-Methyl-estra-1,3,5(10),6-tetraen-1,17β-diol und 4-Methyl-estra-1,3,5(10),6,8-pentaen-1,17β-diol verwendet werden.

7. Arzneimittel, enthaltend mindestens ein nichtestrogenes Derivat des 17α-Estradiols oder des 17β-Estradiols mit antioxidativer Aktivität nach einem der Ansprüche 1 bis 4.

## Claims

1. Non-oestrogenic derivatives of oestradiol having antioxidative activity, of the general formula Ia, 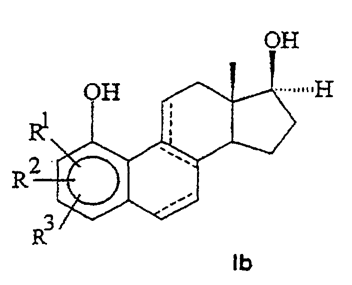
R¹ = H, OH
R², R³ = H, CH₃
which can contain one or two double bonds,
where the hydroxyl groups of the oestradiol derivatives can be present as ethers, esters or sulphamates.

2. Non-oestrogenic derivatives of oestradiol according to Claim 1, namely 4-methyloestra-1,3,5(10)-triene-1,17α-diol, 4-methyloestra-1,3,5(10),6-tetraene-1,17α-diol and 4-methyloestra-1,3,5(10),6,8-pentaene-1,17α-diol.

3. Non-oestrogenic derivatives of 17β-oestradiol having antioxidative activity, of the general formula II, 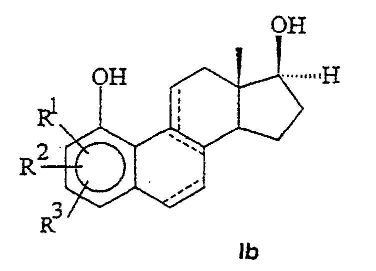
R¹ = H, OH
R², R³ = H, CH₃
Z = (CH₂)ₙAPh
where
n = 0,1
A = a bond for n = 0,1, O, S, Se for n = 1,
which can contain one or two double bonds,
having a 17α substituent which contains a phenyl group Ph,
which is functionalized with 1 or 2 hydroxyl groups and 0 to 2 methyl groups or with a dimethylaminophenyl group and 0 to 2 methyl groups,
where the hydroxyl groups of the 17β-oestradiol derivatives can be present as ethers, esters or sulphamates.

4. Non-oestrogenic derivatives of 17β-oestradiol according to Claim 3, namely 17α-4'-hydroxyphenylmethyl-4-methyloestra-1,3,5(10)-triene-1,17β-diol, 17α-4'-hydroxyphenoxymethyl-4-methyloestra-1,3,5-(10)-triene-1,17β-diol, 17α-4'-hydroxythiophenoxymethyl-4-methyloestra-1,3,5(10)-triene-1,17β-diol, 17α-4'-dimethylaminophenylmethyl-4-methyloestra-1,3,5(10)-triene-1,17β-diol, 17α-3', 5'-dimethyl-4-hydroxyphenylmethyl-4-methyloestra-1,3,5(10)-triene-1,17β-diol, 17α-3',5'-dimethyl4'-hydroxyphenylmethyl-4-methyloestra-1,3,5(10), 6-tetraene-1,17β-diol and 17α-4'-hydroxyphenoxymethyl-4-methyloestra-1,3,5(10), 6-tetraene-1,17β-diol.

5. Use of the non-oestrogenic derivatives of oestradiol and of 17β-oestradiol having antioxidative activity according to one of Claims 1 to 4 and of the non-oestrogenic derivatives of oestradiol having antioxidative activity, of the general formula Ib 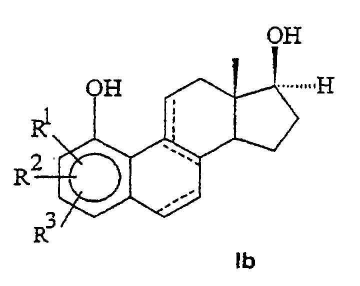
R¹ = H, OH
R², R³ = H, CH₃,
which can contain one or two double bonds,
where the hydroxyl groups of the oestradiol derivatives can be present as ethers, esters or sulphamates, for the production of a medicament for the prophylaxis and therapy of free radical-mediated cell damage.

6. Use according to Claim 5, where the non-oestrogenic derivatives of oestradiol used are 4-methyloestra-1,3,5(10),6-tetraene-1,17β-diol and 4-methyloestra-1,3,5(10),6,8-pentaene-1,17β-diol.

7. Medicaments comprising at least one non-oestrogenic derivative of 17α-oestradiol or of 17β-oestradiol having antioxidative activity according to one of Claims 1 to 4.

## Revendications

1. Dérivés non estrogéniques de l'estradiol à activité anti-oxydante de formule générale Ia, 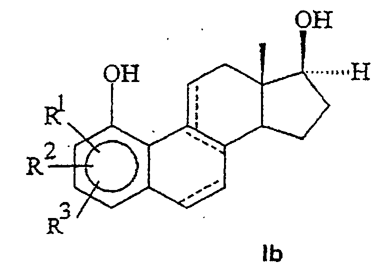
R¹ = H, OH
R², R³ = H, CH₃,
qui peuvent renfermer une à deux doubles liaisons,
les groupes hydroxy des dérivés de l'estradiol pouvant se trouver sous forme d'éthers, d'esters ou de sulfamates.

2. Dérivés non estrogéniques de l'estradiol selon la revendication 1, à savoir le 4-méthyl-estra-1,3,5(10)-triène-1,17α-diol, le 4-méthyl-estra-1,3,5(10),6-tétraène-1,17α-diol et le 4-méthyl-estra-1,3,5(10),6,8-pentaène-1,17α-diol.

3. Dérivés non estrogéniques du 17β-estradiol à activité anti-oxydante de formule générale II, 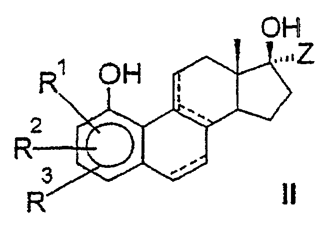
R¹ = H, OH
R², R³ = H, CH₃
Z = (CH₂)ₙAPh,
où
n = 0,1
A = liaison pour n = 0,1, O, S, Se pour n = 1,
qui peuvent renfermer une à deux doubles liaisons,
avec un substituant en 17α renfermant un groupe phényle Ph qui est fonctionnalisé par 1 à 2 groupes hydroxy et 0 à 2 groupes méthyle ou par un groupe diméthylaminophényle et 0 à 2 groupes méthyle,
les groupes hydroxy des dérivés du 17β-estradiol pouvant se présenter sous forme d'éthers, d'esters ou de sulfamates.

4. Dérivés non estrogéniques du 17β-estradiol selon la revendication 3, à savoir le 17α-4'-hydroxy-phénylméthyl-4-méthyl-estra-1,3,5(10)-triène-1,17β-diol, le 17α-4'-hydroxy-phénoxyméthyl-4-méthyl-estra-1,3,5(10)-triène-1,17β-diol, le 17α-4'-hydroxy-thiophénoxyméthyl-4-méthyl-estra-1,3,5(10)-triène-1,17β-diol, le 17α-4'-diméthylamino-phénylméthyl-4-méthyl-estra-1,3,5(10)-triène-1,17β-diol, le 17α-3',5'-diméthyl-4'-hydroxy-phénylméthyl-4-méthyl-estra-1,3,5(10)-triène-1,17β-diol, le 17α-3',5'-diméthyl-4'-hydroxy-phénylméthyl-4-méthyl-estra-1,3,5(10), 6-tétraène-1,17β-diol et le 17α-4'-hydroxy-phénoxyméthyl-4-méthyl-estra-1,3,5(10),6-tétraène-1,17β-diol.

5. Utilisation des dérivés non estrogéniques de l'estradiol et du 17β-estradiol à activité anti-oxydante selon l'une quelconque des revendications 1 à 4 ainsi que des dérivés non estrogéniques de l'estradiol à activité anti-oxydante de formule générale Ib 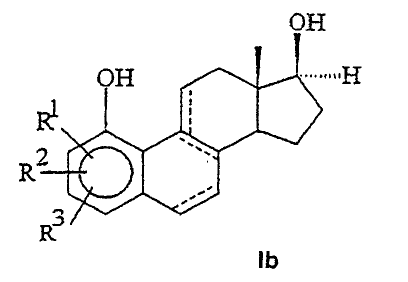
R¹ = H, OH
R², R³ = H, CH₃,
qui peuvent renfermer une à deux doubles liaisons,
les groupes hydroxy des dérivés de l'estradiol pouvant se trouver sous forme d'éthers, d'esters ou de sulfamates, pour la préparation d'un médicament destiné à la prophylaxie et à la thérapie de lésions cellulaires induites par des radicaux.

6. Utilisation selon la revendication 5, en utilisant le 4-méthyl-estra-1,3,5(10),6-tétraène-1,17β-diol et le 4-méthyl-estra-1,3,5(10),6,8-pentaène-1,17β-diol en tant que dérivés non estrogéniques de l'estradiol.

7. Médicament comprenant au moins un dérivé non estrogénique du 17α-estradiol ou du 17β-estradiol à activité anti-oxydante selon l'une quelconque des revendications 1 à 4.
